# EUROPEAN PATENT SPECIFICATION

(11) **EP 2 986 335 B1**
(45) Date of publication and mention of the grant of the patent: **15.08.2018**
(21) Application number: 14716865.2
(22) Date of filing: 14.04.2014
(51) Int. Cl.: A61M 5/315, F16F 3/04, F16F 1/10, A61M 5/20, A61M 5/24, A61M 5/31

(54) **DRUG DELIVERY DEVICE WITH COMPACT POWER UNIT**
ARZNEIMITTELABGABEVORRICHTUNG MIT KOMPAKTER ANTRIEBSEINHEIT
DISPOSITIF D'ADMINISTRATION DE MÉDICAMENT AVEC UNITÉ D'ALIMENTATION COMPACTE

(30) Priority: 19.04.2013 EP 13164533; 22.04.2013 US 201361814514 P
(43) Date of publication of application: 24.02.2016
(73) Proprietor: Novo Nordisk A/S, 2880 Bagsværd (DK)
(72) Inventor: KIILERICH, Ebbe, 2880 Bagsværd (DK)
(86) International application number: PCT/EP2014/057524
(87) International publication number: WO 2014/170267

(56) References cited:
- EP-A2- 1 526 238
- US-A- 3 955 809
- US-A1- 2011 092 905

## Description

### FIELD OF THE INVENTION

The present invention relates to power assisted drug delivery devices, such as e.g. automatic injection devices. The invention also relates to power units useable in such drug delivery devices.

### BACKGROUND OF THE INVENTION

Drug delivery devices, such as injection devices, are widely used for administration of liquid drugs to people in need of therapeutic treatment. Many injection devices are capable of repeated setting and injection of either a fixed or a variable volume of drug upon operation of respective dose setting and injection mechanisms in the device. Some injection devices are adapted to be loaded with a prefilled drug reservoir containing a volume of drug which is sufficient to provide for a number of injectable doses. When the reservoir is empty, the user replaces it with a new one and the injection device can thus be used again and again. Other injection devices are prefilled when delivered to the user and can only be used until the drug reservoir has been emptied, after which the device is discarded. The various injection devices typically expel the drug by advancing a piston in the reservoir using a motion controlled piston rod.

Some injection devices require the user to depress a push button a certain distance towards a housing to thereby manually cause the piston rod to pressurise the reservoir and advance the piston therein for expelling of a selected dose. The force which must be applied to the push button to perform such an operation is typically not insignificant and may cause handling problems for people with reduced finger strength and/or dexterity.

Automatic injection devices offering automatic expelling of a set dose of drug in response to a release of a cocked spring are popular because the spring, once released, provides all the energy needed to complete the injection. Some such devices only require the user to apply a small, short-duration force to trigger the injection. The spring can either be arranged to be strained before each injection, or it can be pre-strained, e.g. by the manufacturer, to deliver energy sufficient to empty the drug reservoir in one or more injections. A spring arrangement of the former type requires the user to provide the energy for straining the spring, a requirement which is viewed as burdensome by some people.

US 2011/0092905 discloses an example of an automatic injection device in which a pre-strained helical spring is adapted to release energy for rotating a drive shaft relative to an injection device body. The rotation of the drive shaft causes a helical displacement of a piston rod and thereby an axial advancement of a piston in a medicated cartridge.

EP 1 526 238 discloses an actuator assembly for vehicle doors and other closures, which assembly comprises a dual spiral spring arrangement, addressing the problem of return mechanisms that have a high failure rate due to fatigue.

US 3,955,809 discloses a dual spiral spring arrangement for induction disk type relays, which spring arrangement has a small spring constant and a large mechanical strength.

WO 2009/105909 (Tecpharma Licensing) discloses an automatic pen-shaped injection device utilising a clock spring to provide energy for activation of the injection mechanism. The clock spring is adapted to be strained during dose setting by rotation of a dose setting button, which means that each time a user sets a dose the dose setting button is rotated against a biasing force of the spring. To avoid this drawback the manufacturer could choose to incorporate a larger dimension clock spring having an increased number of coils to enable storage of an amount of energy sufficient to provide for a complete emptying of the reservoir. However, since the torque deliverable by a clock spring decreases as the spring relaxes it would require a significant size increase of the spring to ensure that sufficient power is available to fully advance the piston in the cartridge. This would compromise the slender configuration of the device and result in a less user-friendly, bulky design.

In view of the above, it is desirable to provide a compact drug delivery mechanism having storage capacity for automatic emptying of a full drug reservoir.

### SUMMARY OF THE INVENTION

It is an object of the invention to eliminate or reduce at least one drawback of the prior art, or to provide a useful alternative to prior art solutions.

In particular, it is an object of the invention to provide a drug delivery device incorporating a power unit which is capable of delivering energy sufficient for occasioning a complete emptying of a full drug container.

In the disclosure of the present invention, aspects and embodiments will be described which will address one or more of the above objects and/or which will address objects apparent from the following text. The scope of the invention is defined in the independent claim. Further embodiments are described in the dependent claims.

In one aspect of the invention, a power unit for a drug delivery device is provided, the power unit comprising a torque transferring structure defining a general axis, a first torque generating structure comprising a first end portion and a second end portion adapted to undergo a first relative angular displacement, the second end portion being rotationally locked to the torque transferring structure at a first axial position, and a second torque generating structure comprising a third end portion and a fourth end portion adapted to undergo a second relative angular displacement, the third end portion being rotationally locked to the torque transferring structure at a second axial position which is different from the first axial position.

The first torque generating structure may be adapted to store energy when the first end portion undergoes relative rotation in a first direction with respect to the second end portion and to release energy when the first end portion undergoes reverse relative rotation with respect to the second end portion. The second torque generating structure may be adapted to store energy when the fourth end portion undergoes relative rotation in a second direction with respect to the third end portion and to release energy when the fourth end portion undergoes reverse relative rotation with respect to the third end portion. The first direction and the second direction may be the same or opposite directions.

The first torque generating structure and the second torque generating structure may in particular be respective first and second spiral spring members, such as e.g. planar spiral spring members or helical spring members. At least one of the first spiral spring member and the second spiral spring member may be wound concentrically about the torque transferring structure. Alternatively, or additionally, at least one of the first spiral spring member and the second spiral spring member may be surrounded by a portion of the torque transferring structure. The first spiral spring member may be in a relaxed state or in a tensed state, and the second spiral spring member may be in a relaxed state or in a tensed state.

In case the first spiral spring member is in a tensed state the first relative angular displacement releases energy stored in the first spiral spring member, and, similarly, in case the second spiral spring member is in a tensed state the second relative angular displacement releases energy stored in the second spiral spring member.

In particular embodiments of the invention, the first relative angular displacement and the second relative angular displacement are equal, or substantially equal, e.g. obtained by two spiral spring members having identical, or substantially identical dimensions and identical, or substantially identical, material properties.

In another aspect, the invention provides a power unit for a drug delivery device comprising a first interface portion and a second interface portion, the power unit comprising a torque transferring structure arrangeable for rotation relative to the first interface portion and the second interface portion, first torque applying means adapted to operate between the first interface portion and the torque transferring structure to induce a relative angular displacement between the torque transferring structure and the first interface portion, and second torque applying means adapted to operate between the torque transferring structure and the second interface portion to induce a relative angular displacement between the torque transferring structure and the second interface portion.

The operation of such a power unit will result in a total relative angular displacement between the second interface portion and the first interface portion which equals the sum of the relative angular displacement between the torque transferring structure and the first interface portion and the relative angular displacement between the torque transferring structure and the second interface portion. In particular, in case the first interface portion can be considered rotationally fixed, the first torque applying means may be adapted to angularly displace the torque transferring structure in one rotational direction relative to the first interface portion, while the second torque applying means may be adapted to angularly displace the second interface portion in said one rotational direction relative to the torque transferring structure. Thereby, the second interface portion will undergo a total angular displacement in said one direction relative to the first interface portion which equals the sum of the angular displacement of the torque transferring structure relative to the first interface portion and the angular displacement of the second interface portion relative to the torque transferring structure.

The drug delivery device may be of the type in which a relative angular displacement between the first interface portion and the second interface portion causes a volume reduction of a drug reservoir, i.e. an expelling of drug therefrom. Alternatively, or additionally, the drug delivery device may be of the type in which a relative angular displacement between the first interface portion and the second interface portion causes a dose to be set, or an operational state of the drug delivery device to change.

The first torque applying means comprises a clock spring. Similarly, but not necessarily identically, the second torque applying means also comprises a clock spring. A power unit according to the present invention may comprise two or more serially arranged spring members, at least one of the spring members being couplable to the first interface portion of the drug delivery device and at least one other of the spring members being couplable to the second interface portion of the drug delivery device which when undergoing relative motion with respect to the first interface portion causes a change of state of the drug delivery device, e.g. a setting of a dose, or an expelling of drug therefrom.

In yet another aspect of the invention a power unit for a drug delivery device is provided, the power unit extending along a general axis and comprising a first spiral spring member, e.g. a first planar torque spring member, comprising a first coil end portion and a second coil end portion, and a second spiral spring member, e.g. a second planar torque spring member, comprising a third coil end portion and a fourth coil end portion. The first coil end portion is adapted for rotational fixation to a first interface portion of the drug delivery device, and the fourth coil end portion is adapted for rotational fixation to a second interface portion of the drug delivery device, which is rotatable relative to the first interface portion. The third coil end portion is rotationally locked with respect to the second coil end portion but axially offset therefrom, and the power unit is adapted to release energy for inducing relative rotation between the first interface portion and the second interface portion, e.g. for inducing a rotation of the second interface portion with respect to the first interface portion.

In the present context, when a coil end portion is rotationally fixed to an interface portion, such as e.g. a drug delivery device portion, the specific coil end portion and the specific interface portion are incapable of undergoing at least one relative angular displacement (i.e. they are either both angularly fixed or they are bound to rotate jointly in at least one direction).

The first coil end portion may further be adapted for translational fixation to the first interface portion. Alternatively, the first coil end portion may be adapted for relative translational movement with respect to the first interface portion. Likewise, the fourth coil end portion may further be adapted for translational fixation to, or relative translational movement with respect to, the second interface portion.

A power unit according to any of the above aspects of the invention can be realised as a compact construction with the capacity to deliver a number of revolutions to the second interface portion which are sufficient to collapse a dedicated drug reservoir, as well as to apply a relatively constant torque to the second interface portion over its entire working range. Especially, the power unit can be realised as a slender construction suitable for use in a pen type injection device.

Although the power unit is suitable for being pre-strained to store energy sufficient to empty a full reservoir, it may nevertheless alternatively be adapted to be strained before each activation of the expelling mechanism. In case the reservoir contains more drug than the expelling mechanism allows to expel in one go the coil spring dimensions may be chosen such as to enable the power unit to store energy sufficient for delivery of the maximum expellable dose instead of for delivery of a volume corresponding to the capacity of the reservoir, in which case the power unit can be made even slimmer.

The first spiral spring member and the second spiral spring member may be respective clock springs. In that case, the second coil end portion and the third coil end portion may be respective inner coil end portions, and the first coil end portion and the fourth coil end portion may be respective outer coil end portions. The second coil end portion and the third coil end portion may be rotationally locked to an axially extending, e.g. torsionally rigid, structure, which axially extending structure may function as a central spine, enabling a particularly slender configuration of the power unit.

The first spiral spring member and the second spiral spring member may be wound in opposite directions about the axially extending structure to enable maximum additive torque transmission and angular displacement between the first coil end portion and the fourth coil end portion. If the first spiral spring member and the second spiral spring member are arranged concentrically about the general axis and are axially offset, and if, further, they are wound in opposite directions a high capacity compact power unit may be provided.

Alternatively, the first spiral spring member and the second spiral spring member may be wound in the same direction, in which case the second coil end portion and the fourth coil end portion may be respective inner coil end portions, while the first coil end portion and the third coil end portion are respective outer coil end portions, or vice versa. The axially extending structure may then be configured to interface with, respectively, an inner coil end portion and an outer coil end portion of two concentrically arranged springs. This will allow for use of the power unit in drug delivery devices where e.g. the second interface portion is suited for being surrounded by the second spiral spring member.

The respective coil end portions may be provided with separate means for engagement with the respective first and second interface portions or the axially extending structure. Alternatively, the coil end portions may respectively be formed to receive, or to be received in, the respective first and second interface portions or the axially extending structure.

In particular embodiments, the first spiral spring member and the second spiral spring member are identical, or at least substantially identical, in dimensions and material properties, whereby their individual capacities are equal, or at least substantially equal.

The power unit may e.g. comprise a spring assembly comprising the first spiral spring member, the second spiral spring member, and the axially extending structure. The axially extending structure may comprise a first shaft portion and a second shaft portion, the first spiral spring member being wound about the first shaft portion and the second coil end portion being attached to the first shaft portion, and the second spiral spring member being wound about the second shaft portion and the third coil end portion being attached to the second shaft portion. The first shaft portion and the second shaft portion may be concentrically arranged, and they may be axially fixed with respect to one another, e.g. provided as a single element or as two elements separated by a radially extending structure, such as a disc. Alternatively, the axially extending structure may comprise a shaft portion and a drum portion, one of the first spiral spring member and the second spiral spring member being attached to and wound about the shaft portion, and the other of the first spiral spring member and the second spiral spring member being arranged within, and attached to, the drum portion. The axially extending structure may be adapted for rotational mounting in the drug delivery device and may comprise an axially extending central passage for reception of e.g. a portion of the drug delivery device.

The axially extending structure may be torsionally flexible. In particular, the axially extending structure may comprise a first anchor part, to which the second coil end portion may be fixed, and a second anchor part, to which the third coil end portion may be fixed, and the first anchor part and the second anchor part may be biased towards relative rotation. For example, the axially extending structure may comprise a torque generating structure, such as a spiral spring member, acting between the first anchor part and the second anchor part. Thereby, the axially extending structure itself is capable of contribution to the total relative angular displacement between the first coil end portion and the fourth coil end portion, enabling the provision of an even higher capacity power unit.

In practice, the axially extending structure may be realised with multiple anchor parts and appurtenant multiple torque generating structures arranged in series between the first anchor part and the second anchor part to provide individual contributions to a total relative angular displacement between the first anchor part and the second anchor part. Power units can thus be provided for emptying of large volume reservoirs without compromising on the attractive slender configuration of the drug delivery device.

The power unit may alternatively comprise a unitary spring element comprising the first spiral spring member, the second spiral spring member, and the axially extending structure. In that case, a single piece of material, such as a thin metal sheet, may be formed with an axially extending central portion and two lateral strips extending from opposite sides thereof. The central portion itself may be bendable and the strips may be bendable about the central portion, allowing concentric coils to be produced. Thereby, a very simple and inexpensive power unit may be provided.

In a further aspect of the invention a drug delivery device is provided comprising a power unit as described in connection with any one of the above aspects and embodiments.

For example, a drug delivery device may be provided comprising a first interface portion, a second interface portion, and a power unit releasable to generate and transfer a torque between the first interface portion and the second interface portion to induce a relative rotational movement between the first interface portion and the second interface portion.

In particular, a drug delivery device may be provided comprising a first interface portion, an expelling mechanism operable to deliver a dose of substance from a reservoir, the expelling mechanism comprising a second interface portion, the second interface portion being capable of undergoing relative rotation with respect to the first interface portion to cause the dose of substance to be delivered from the reservoir, and a power unit adapted to release energy for inducing relative rotation between the first interface portion and the second interface portion, the power unit extending along a longitudinal axis and comprising a) a first spiral spring member comprising a first inner coil end portion and a first outer coil end portion, and b) a second spiral spring member comprising a second inner coil end portion and a second outer coil end portion, wherein one of the first inner coil end portion and the first outer coil end portion is rotationally fixed to the first interface portion, and one of the second inner coil end portion and the second outer coil end portion is rotationally fixed to the second interface portion, and wherein the other of the first inner coil end portion and the first outer coil end portion is rotationally locked with respect to, and axially offset from, the other of the second inner coil end portion and the second outer coil end portion.

The drug delivery device may further comprise a housing for accommodating at least a portion of the power unit and to which the reservoir may be at least translationally fixed during use. The first interface portion may be a portion of the housing, or it may be rotationally fixed with respect to the housing, at least when the power unit releases energy. In either case a release of energy from the power unit will then cause a rotation of the second interface portion relative to the housing.

The expelling mechanism may further comprise an actuator, such as e.g. a piston rod, displaceable to reduce a volume of the reservoir, and transmission means for transmitting a rotational output of the second interface portion to the actuator. The transmission means may comprise a sleeve member rotationally and translationally locked to the second interface portion, a guide member rotationally mounted in the housing and configured for engagement with the sleeve member during release of the power unit, the guide member being rotationally locked with respect to the actuator, and a nut member stationarily arranged in the housing and threadedly engaged with the actuator. During release of the power unit the sleeve member and the guide member are then brought into engagement and the rotation of the second interface portion is thus transferred to the actuator which advances distally through the housing to pressurise the reservoir.

The drug delivery device may further comprise an activation button operable by application of a user controlled activation force to activate the expelling mechanism. The activation button may be configured to move between an activatable position in which the expelling mechanism is at rest and an activated position in which the expelling mechanism is activated by the power unit. During a movement from the activatable position to the activated position the activation button may cause the sleeve member to engage with the guide member, and during a movement from the activated position to the activatable position the activation button may cause the sleeve member to disengage from the guide member.

The activation button may be biased towards the activatable position, e.g. by means of a suitable spring, in which case an on-going expelling of a dose may be selectively paused by discontinuation of the activation force.

The axially extending structure may form an axial passage, and the activation button may comprise a distally pointed pin structure which extends through the passage and thereby constitutes a hub for the power unit. The activation button may be translationally fixed with respect to the second interface portion, e.g. by engagement between the pin structure and a portion of the sleeve member. Any translational movement of the activation button will thereby be transferred to the second interface portion and the sleeve member, allowing for both a resulting coupling of the sleeve member to the guide member and subsequent release of the second interface portion from a retaining structure and a resulting re-engagement of the second interface portion with the retaining structure and subsequent decoupling of the sleeve member from the guide member.

The drug delivery device may further comprise a dose setting mechanism for selective setting of a dose to be delivered by subsequent activation of the expelling mechanism. The dose setting mechanism may comprise a dose dial adapted for rotation relative to the housing for setting of the dose, and a dose display for indicating the set dose. Particularly, the dose display may comprise a scale drum having numerals attached thereto, e.g. printed thereon, and a window through which a portion of the scale drum is visible.

The dose dial may further be adapted for rotational fixation with respect to the housing during expelling of a set dose. For example, the dose dial may be rotationally locked to the activation button, and the activation button may be adapted to rotationally interlock with the housing during a movement from the activatable position to the activated position and rotationally disengage from the housing during a movement from the activated position to the activatable position.

The scale drum may be threadedly engaged with the housing and configured to move helically in one axial direction during an increase of the set dose and in the opposite axial direction during a decrease of the set dose. The scale drum may further be rotationally locked with respect to the second interface portion, whereby a direct coupling between the dose setting mechanism and the expelling mechanism is provided in the sense that every time the second interface portion rotates the scale drum performs a correlated movement in the housing to define either the dose set or the dose expelled from the reservoir, depending on whether the second interface portion rotates jointly with or relative to the dose dial.

The first interface portion may be a portion of the dose dial, preferably an inner portion of the dose dial such as an interior member. This will enable the power unit to be non-distortedly rotated along with the dose dial during dose setting and to release energy for maximum displacement of e.g. the second outer coil end portion relative to the housing, thereby providing for maximum transmission to the expelling mechanism, during dose delivery. At least a portion of the power unit may thus be contained within the dose dial, whereby it can be ensured that the power unit construction will add insignificantly little or nothing to the total axial dimension of a drug delivery device in which it is incorporated.

The drug delivery device may be any kind of device capable of parenteral drug delivery, such as e.g. an injection device, an infusion device, or an inhalation device. In particular, the drug delivery device may be a handheld injection device (e.g. for self-administration of a glucose regulating agent), such as a pen injector, i.e. an injection device having a shape which resembles that of a fountain pen. Further, the drug delivery device may comprise an integrated drug reservoir, or it may be adapted to receive a separately provided drug reservoir.

A drug delivery device according to the present invention may comprise two or more serially arranged power units to increase the storage capacity for expelling drug from a reservoir.

In the present specification, reference to a certain aspect or a certain embodiment (e.g. "an aspect", "a first aspect", "one embodiment", "an exemplary embodiment", or the like) signifies that a particular feature, structure, or characteristic described in connection with the respective aspect or embodiment is included in, or inherent of, at least that one aspect or embodiment of the invention, but not necessarily in/of all aspects or embodiments of the invention. It is emphasized, however, that any combination of the various features, structures and/or characteristics described in relation to the invention is encompassed by the invention unless expressly stated herein or clearly contradicted by context.

The use of any and all examples, or exemplary language (e.g., such as, etc.), in the text is intended to merely illuminate the invention and does not pose a limitation on the scope of the same, unless otherwise claimed. Further, no language or wording in the specification should be construed as indicating any non-claimed element as essential to the practice of the invention.

### BRIEF DESCRIPTION OF THE DRAWINGS

In the following the invention will be further described with references to the drawings, wherein
Fig. 1 is an exploded perspective view of a drug delivery device according to an embodiment of the invention,
Fig. 2 is a longitudinal sectional view of the drug delivery device of Fig. 1, in a pre-use state,
Fig. 3 is an exploded perspective view of the power unit incorporated in the drug delivery device of Fig. 1,
Fig. 4a is a top view of a non-coiled power unit suitable for use, in a coiled state, in the drug delivery device of Fig. 1,
Fig. 4b is a perspective view of the power unit of Fig. 4a, in a coiled state,
Fig. 5 is an exploded perspective view of a power unit suitable for use in a drug delivery device according to an alternative embodiment of the invention, and
Fig. 6 is an exploded perspective view of a power unit also suitable for use in the drug delivery device of Fig. 1.

In the figures like structures are mainly identified by like reference numerals.

### DESCRIPTION OF EXEMPLARY EMBODIMENTS

When in the following relative expressions, such as "clockwise" and "counter-clockwise", are used, these refer to the appended figures and not necessarily to an actual situation of use. The shown figures are schematic representations for which reason the configuration of the different structures as well as their relative dimensions are intended to serve illustrative purposes only.

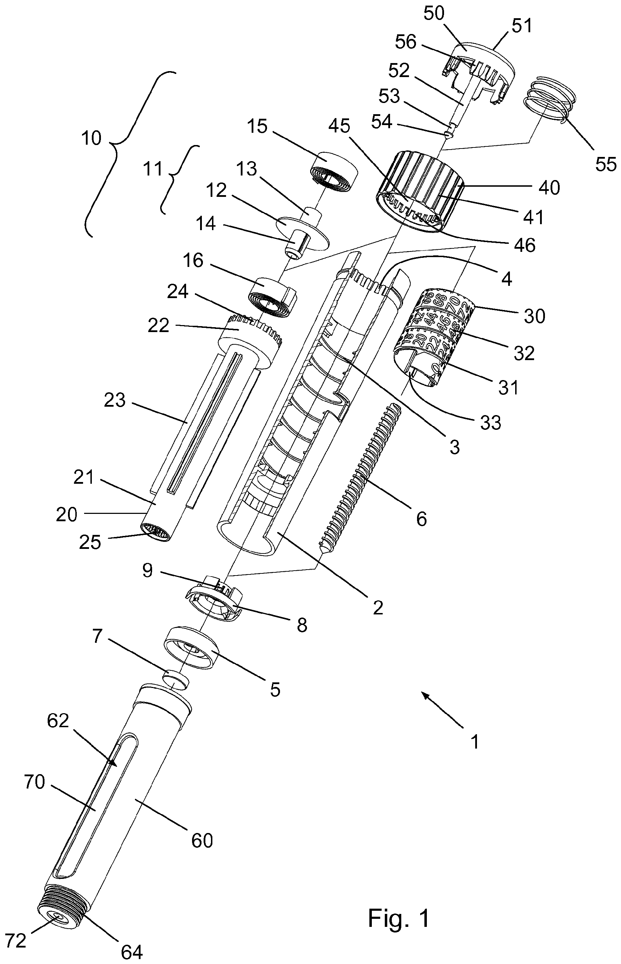

Fig. 1 shows a pen-type injection device 1 for delivery of a liquid drug substance, such as e.g. insulin or glp-1 for the treatment of diabetes. The injection device 1 comprises a longitudinal housing 2 which defines a general axis. The housing 2 has an interior thread 3 as well as a plurality of spaced apart axial grooves 4 distributed along an inner circumference at its proximal end. A cartridge holder 60 accommodating a cartridge 70 is removably or irremovably attached to the distal end portion of the housing 2, such as e.g. by gluing, snap fitting, bayonet coupling or the like. At its distal end portion the cartridge holder 60 is provided with a needle hub interface 64 for reception of an injection needle unit (not shown). The contents of the cartridge 70 can be inspected through an elongated window 62 in the cartridge holder 60, and access to the interior of the cartridge 70 can be obtained by penetration of a resealable septum 72.

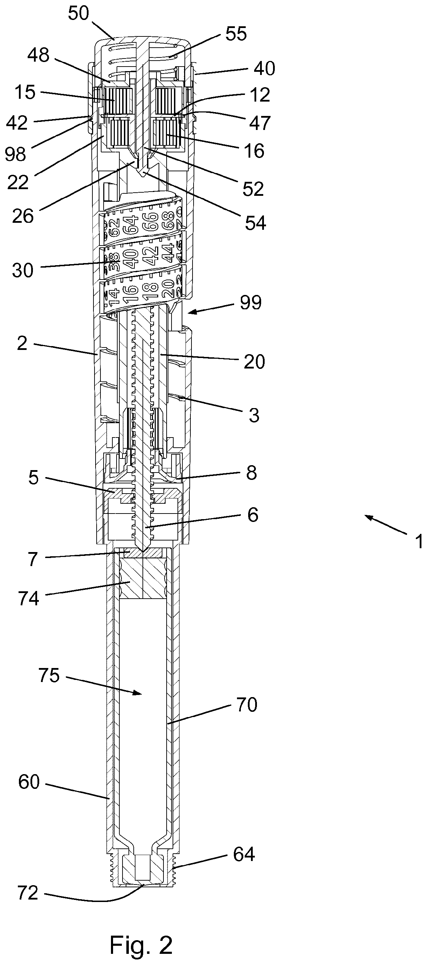

A threaded piston rod 6 arranged in the housing 2 extends through a piston rod guide 8 and a nut 5 and is at its distal end configured for abutment with a piston rod foot 7 which rests on a proximal end portion of a piston 74 (see Fig. 2). The piston rod 6 is splined to the piston rod guide 8, whereby the piston rod 6 and the piston rod guide 8 are capable of relative axial movement but not of relative rotation. The piston rod 6 is further threadedly received in the nut 5. The nut 5 is translationally as well as rotationally fixed in the housing 2, whereas the piston rod guide 8 is translationally fixed in, but rotatable counter-clockwise with respect to, the housing 2.

The housing 2 further accommodates a driver 20 which serves as both a spring fixture and a transmission element, as will be apparent from the below. The driver 20 comprises an axial sleeve 21, surrounding at least a portion of the piston rod 6, and a proximal cup 22. A plurality of longitudinal ribs 23 are provided on the exterior surface of the sleeve 21 and a plurality of spaced apart axial grooves 24 are distributed along the periphery of the cup 22 at its proximal end. At the interior distal end portion of the sleeve 21 a plurality of circumferentially spaced apart axial grooves 25 are provided. The grooves 25 are intended for engagement with corresponding ridges 9 provided on the piston rod guide 8, at a specific axial position of the driver 20 in the housing 2, so as to provide for joint rotation of the driver 20 and the piston rod guide 8 relative to the housing 2.

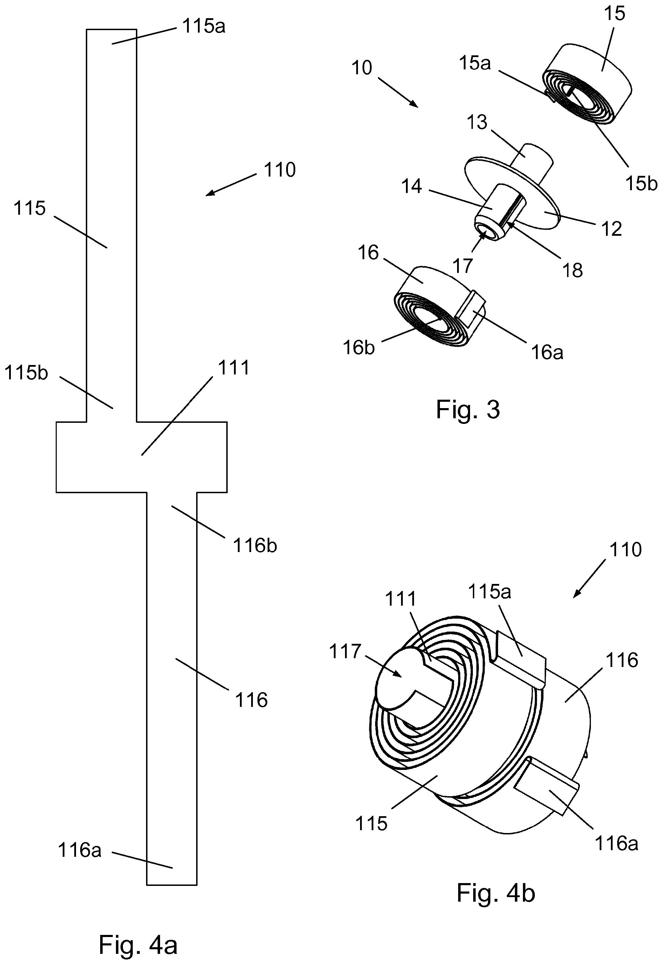

The injection device 1 also comprises a spring assembly 10 for delivering energy to expel the drug from the cartridge 70 when an injection needle unit is attached to the needle hub interface 64. The spring assembly 10 comprises a rotor 11 comprising a proximal shaft 13, a distal shaft 14 and a dividing plate 12, a proximal spiral spring 15, an inner coil end portion 15b (see Fig. 3) of which being attached to the proximal shaft 13, and a distal spiral spring 16, an inner coil end portion 16b (see Fig. 3) of which being attached to the distal shaft 14. Seen from a proximal perspective the proximal spring 15 is wound counter-clockwise about the proximal shaft 13 and the distal spring 16 is wound clockwise about the distal shaft 14. Further, the two springs 15, 16 have substantially equal dimensions and moduli of elasticity, and they are thus capable of providing additive torques and angular displacements of comparable magnitudes, with the rotor 11 as a floating torque transferring intermediate medium. An outer coil end portion 16a (see Fig. 3) of the distal spiral spring 16 is attached to an inner portion of the cup 22 so as to provide for joint rotation of the outer coil end portion 16a and the driver 20.

An outer coil end portion 15a (see Fig. 3) of the proximal spiral spring 15 is attached to an interior member 45 of a dose dial 40, providing rotational fixation of the outer coil end portion 15a to the dose dial 40. The dose dial 40 is user operable to set a desired dose to be delivered from the cartridge 70, and it has axially extending surface corrugations 41, providing for easy dialling in both directions. A plurality of axially extending teeth 46 are arranged circumferentially at the distal end portion of the interior member 45 for engagement with the grooves 24 in the cup 22 during dose setting. Hence, when a dose is set by turning of the dose dial 40 a certain number of degrees the driver 20 rotates and angularly displaces the same number of degrees.

Occupied in the housing 2 is also a scale drum 30, having a plurality of numerals 32 printed thereon along a helical path. Each numeral 32 represents a specific dose to be delivered and is correlated with the distance the piston rod 6 travels during an expelling of the drug. The scale drum 30 also has an exterior thread 31 which cooperates with the interior thread 3, and a number of axially extending tracks 33 providing for a splined relationship with the ribs 23 on the sleeve 21. Any rotation of the driver 20 is therefore transferred to the scale drum 30, and vice versa.

A user operable injection button 50 is arranged at the proximal end portion of the housing 2. The injection button 50 has a proximal push surface 51 for receiving a finger and a distally extending pin body 52 which passes through the rotor 11. Distally of the rotor 11 the pin body 52 ends in a neck 53 and a spear head 54. Also, axially extending ridges 56 are adapted for reception in the grooves 4, securing rotational fixation of the injection button 50 to the housing 2, when the injection button 50 is depressed into the housing 2 during dose injection. The injection button 50 is biased away from the housing 2 by a button spring 55. This means that when no force is applied to the push surface 51 the ridges 56 are disengaged from the grooves 4, and the injection button 50 is allowed to rotate relative to the housing 2. The injection button 50 and the dose dial 40 are rotationally locked to one another at all times.

Fig. 2 is a longitudinal sectional view of the injection device 1, the scale drum 30 being shown non-sectioned. The injection device 1 is in a pre-use state where no dose is yet dialled. In this state a dose window 99 in the housing 2 displays the numeral "0" (not visible) to the user. The cartridge 70 is closed at its distal end by the septum 72 and at its proximal end by the piston 74, providing a chamber 75 in which the drug (not shown) is contained.

The dose dial 40 has an internal annular groove 42 which engages with a circumferential ridge 98 on the housing 2, translationally fixating the dose dial 40 to the housing 2. Also, the interior member 45 has an internal annular groove 47 which engages with the plate 12 and thereby serves as a rotational support for the rotor 11 in the dose dial 40. The interior member 45 further has a top portion 48 on which the button spring 55 rests.

Fig. 2 also shows the pin body 52 extending through the rotor 11. An interior constriction 26 at the proximal portion of the sleeve 21 retains the pin body 52 at the neck 53, thereby translationally fixating the injection button 50 to the driver 20.

Fig. 3 is an enlarged exploded view of the spring assembly 10. It shows a longitudinal slot 18 in the distal shaft 14 in which the inner coil end portion 16b is inserted, securing joint rotation of the rotor 11 and the inner coil end portion 16b. A similar slot (not visible) is provided in the proximal shaft 13 for securing joint rotation of the rotor 11 and the inner coil end portion 15b.

Figs. 4a and 4b present an alternative embodiment of a power unit according to the present invention. In this embodiment a spring unit 110 is provided comprising two spiral springs formed from a single piece of material. As can be seen from fig. 4a the spring unit 110 comprises an axial spine 111 and two lateral branches 115, 116 extending in opposite directions therefrom. The lateral branches 115, 116 are capable of being coiled about the axial spine 111, as seen in Fig. 4b, and they comprise respective inner coil end portions 115b, 116b and respective outer coil end portions 115a, 116a prepared for engagement with respective first and second interface portions of a drug delivery device, e.g. as described above in connection with the injection device 1.

The operation of the injection device 1 and the spring assembly 10 will now be described. With reference to Fig. 2, a user wishing to perform an injection with the injection device 1 initially attaches a suitable injection needle unit (not shown) to the needle hub interface 64, whereby the septum 72 will be transpierced and fluid communication to the chamber 75 established. The user may then, in a conventional manner, perform an air-shot to prime the system and eliminate any air pockets in the delivery line. The desired dose to be injected is set by turning the dose dial 40 clockwise (when seen from the top of the figure) a certain number of degrees about the general axis of the injection device 1 until the corresponding numeral 32 appears in the window 99. The turning of the dose dial 40 causes a rotation of the driver 20, due to the engagement between the teeth 46 and the grooves 24, and the scale drum 30, due to the engagement between the ribs 23 and the tracks 33. The induced rotation of the scale drum 30 relative to the housing 2 will cause the thread 31 to move along the interior thread 3, displacing the scale drum 30 helically downwards in the housing 2 from a top position. In case the user for some reason dials a larger dose than the desired dose the turning of the dose dial 40 can be reversed to adjust the dose accordingly. In that respect, a counter-clockwise rotation of the dose dial 40 will merely cause the scale drum 30 to move back upwards in the housing 2.

In this dose setting state of the injection device 1 the cup 22 is firmly coupled to the interior member 45 due to the injection button 50 being biased proximally by the button spring 55, the spear head 54 consequently exerting a proximally directed force to the constriction 26. Thereby, the driver 20 is held in a certain axial position in which it is decoupled from the piston rod guide 8, securing that no movements are transferred to the piston rod 6.

Since the outer coil end portion 16a is rotationally fixated to the cup 22 and the outer coil end portion 15a is rotationally fixated to the interior member 45 of the dose dial 40 the entire spring assembly 10 undergoes a non-deformable rotation, slaved by the dose dial 40 and the driver 20, during dose setting. In this embodiment, the spring assembly 10 is fully pre-tensed, i.e. the proximal spiral spring 15 and the distal spiral spring 16 are together capable of delivering an angular displacement and a torque to the injection mechanism which are sufficient to empty the cartridge 70, over one or more injections.

To inject the set dose the user depresses the injection button 50 into the housing 2 against the biasing force of the button spring 55. This initially causes the ridges 56 to slide into engagement with the grooves 4, thereby rotationally locking the injection button 50 to the housing 2. Since the dose dial 40 is rotationally locked with respect to the injection button 50, the depression of the injection button 50 also initially results in the dose dial 40 becoming rotationally locked to the housing 2.

Any axial movement of the injection button 50 is transferred to the driver 20 via the interaction between the pin body 52 and the constriction 26. Hence, the depression of the injection button 50 subsequently results in the grooves 25 sliding into engagement with the ridges 9, rotationally locking the driver 20 to the piston rod guide 8, and, finally, in the grooves 24 sliding out of engagement with the teeth 46, decoupling the cup 22 from the interior member 45 and releasing the spring assembly 10.

When the driver 20 is thus no longer engaged with the dose dial 40 the pre-tensed spring assembly 10 is free to deliver energy to the injection mechanism. In this dose injection state of the injection device 1 since the dose dial 40 is both translationally and rotationally locked to the housing 2 when the injection button 50 is depressed the outer coil end portion 15a is immovable relative to the housing 2. The partial uncoiling of the proximal spiral spring 15 during the energy release thus leads the inner coil end portion 15b to rotate the proximal shaft 13 and thereby also the plate 12 and the distal shaft 14. Notably, this rotation is counter-clockwise, i.e. opposite to the rotation of the dose dial 40 when the dose is increased during dose setting. Because the inner coil end portion 16b of the distal spiral spring 16 is rotationally fixed to the distal shaft 14 it experiences an angular displacement, θ₁, which corresponds to the angular displacement of the rotor 11 during the partial uncoiling of the proximal spiral spring 15. The distal spiral spring 16 itself, however, also partially uncoils and contributes to the total angular displacement of the outer coil end portion 16a by an angular displacement, θ₂, of the outer coil end portion 16a relative to the distal shaft 14. The outer coil end portion 16a, and thereby the cup 22, thus undergoes a total angular displacement of θ₁ + θ₂ relative to the housing 2.

The counter-clockwise rotation of the driver 20 occasions two simultaneous events. Due to the established engagement between the grooves 25 and the ridges 9 the piston rod guide 8 is forced to co-rotate with the sleeve 21, and, due to the splined relationship between the piston rod guide 8 and the piston rod 6, so is the piston rod 6. The threaded engagement between the piston rod 6 and the nut 5 now causes the piston rod to advance axially relative to the housing 2 and push the piston 74 distally in the cartridge 70, whereby drug is delivered through the needle (not shown). Further, the engagement between the ribs 23 and the tracks 33 causes a counter-clockwise rotation of the scale drum 30, which leads to the thread 31 moving back along the interior thread 3, displacing the scale drum 30 helically upwards in the housing 2 towards the top position.

The expelling of drug from the cartridge 70 continues until the scale drum 30 reaches the top position, in which "0" is displayed in the window 99. In the top position the scale drum 30 can no longer rotate counter-clockwise, consequently bringing the cup 22 to a halt and preventing the spring assembly 10 from releasing further energy. When the user subsequently removes the finger from the push surface 51 the button spring 55 relaxes and lifts the injection button 50 back to its pre-use position. This causes the spear head 54 to lift the cup 22 back into engagement with the interior member 45, thereby decoupling the piston rod guide 8 from the driver 20 and locking the spring assembly 10. The injection device 1 is now ready for a new dose setting procedure.

The biased injection button 50 also provides for optional pausing of an injection. During an injection, in case the user removes her/his finger from the push surface 51 before the scale drum 30 has returned to the top position, the button spring 55 will instantly lift the cup 22, whereby the grooves 24 will engage with the teeth 46 and lock the spring assembly 10 against further rotation. The scale drum 30 will then remain in its current position and the piston rod 6 will be stationary until the injection button 50 is again depressed into the housing 2.

As the user expels doses of drug from the injection device 1, the respective angular deflections of the outer coil end portions 15a, 16a gradually decrease, reducing the potential energy in the spring assembly 10. However, the system comprised by the injection mechanism, the cartridge 70, the injection needle (not shown), and the spring assembly 10 is dimensioned to enable delivery of the entire dispensable amount of drug in the chamber 75 with sufficient force to a specified target site in the body.

The injection device 1 could alternatively be designed to include the spring unit 110 instead of the spring assembly 10. In that case the coiled branches 115, 116 could be occupied in dedicated respective proximal and distal spring housings (not shown), which spring housings could be splined to the dose dial 40. During depression of the injection button 50 the distal spring housing would disengage from the dose dial 40 and transfer a torque from the partially uncoiling branches 115, 116 to the injection mechanism in a manner similar to what is described in the above.

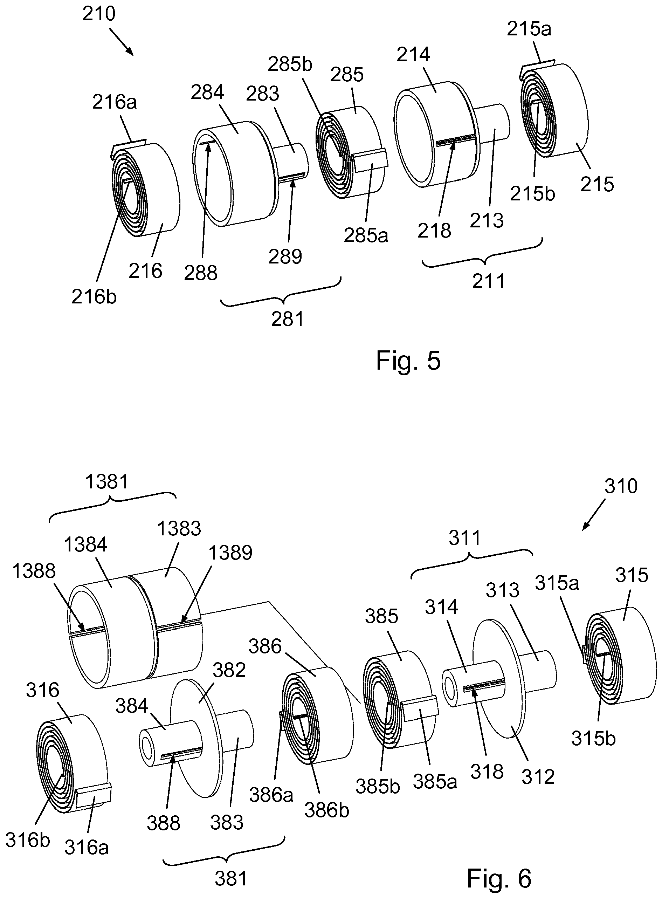

Fig. 5 is an exploded view of an alternative spring assembly 210 having three serially arranged spiral springs. The spring assembly 210 comprises a proximal spiral spring 215 and a distal spiral spring 216 being operatively coupled via a proximal rotor 211, a distal rotor 281, and a central spiral spring 285. The proximal rotor 211 comprises a hollow shaft portion 213 and a drum portion 214. Similarly, the distal rotor 281 comprises a hollow shaft portion 283 and a drum portion 284.

The proximal spiral spring 215 has an outer coil end portion 215a adapted for rotational fixation to a portion of a drug delivery device which is stationary at least during drug delivery, and an inner coil end portion 215b which is rotationally fixed to the shaft portion 213 in a manner similar to the fixation of the inner coil end portion 15b to the proximal shaft portion 13. The central spiral spring 285 has an outer coil end portion 285a which is inserted in a slot 218 in the drum portion 214, securing joint rotation of the rotor 211 and the outer coil end portion 285a, and an inner coil end portion 285b which is inserted in a slot 289 in the shaft portion 283, securing joint rotation of the rotor 281 and the inner coil end portion 285b. The central spiral spring 285 is thus both occupied within the drum portion 214 and coiled about the shaft portion 283. The distal spiral spring 216 has an outer coil end portion 216a which is inserted in a slot 288 in the drum portion 284, securing joint rotation of the rotor 281 and the outer coil end portion 216a, and an inner coil end portion 216b which is adapted for rotational fixation to a portion of the drug delivery device which when rotated with respect to the stationary portion causes e.g. an expelling of a dose of the drug.

The spring assembly 210 could e.g. be used in the injection device 1 instead of the spring assembly 10, provided that the driver 20 was re-designed to rotationally engage with the inner coil end portion 216b. If, for example, the constructional features of the respective spiral springs 215, 216, 285 were identical to those of the respective spiral springs 15, 16 the spring assembly 210 would have a higher capacity than the spring assembly 10 due to the presence of the additional central spiral spring 285, and the spring assembly 210 would consequently be able to provide a larger total angular displacement to the injection mechanism.

In fact, the assembly part consisting of the proximal rotor 211, the proximal spiral spring 215, and the central spiral spring 285 constitutes in itself an alternative power unit to the spring assembly 10, where, notably, the two spiral springs have the same winding direction.

Fig. 6 is an exploded view of a further alternative spring assembly 310 comprising four serially arranged spiral springs. The spring assembly 310 comprises a proximal spring assembly consisting of a proximal spiral spring 315, a proximal rotor 311 and a first central spiral spring 385, a distal spring assembly consisting of a distal spiral spring 316, a distal rotor 381, and a second central spiral spring 386, and a central rotor 1381 operatively coupling the proximal spring assembly and the distal spring assembly. The proximal rotor 311 comprises a hollow proximal shaft 313 and a hollow distal shaft 314 separated by a transversal dividing plate 312. Similarly, the distal rotor 381 comprises a hollow proximal shaft 383 and a hollow distal shaft 384 separated by a transversal dividing plate 382. The central rotor 1381 comprises a proximal drum portion 1383 and a distal drum portion 1384 which are incapable of relative rotation. Also, both the proximal rotor 311 and the distal rotor 381 are torsionally rigid structures.

The proximal spiral spring 315 has an outer coil end portion 315a adapted for attachment to a portion of a drug delivery device which is stationary during drug delivery, and an inner coil end portion 315b which is rotationally fixed to the proximal shaft 313. The first central spiral spring 385 has an inner coil end portion 385b which is inserted in a slot 318 in the distal shaft 314, securing joint rotation of the inner coil end portion 385b and the proximal rotor 311, and an outer coil end portion 385a which is inserted in a slot 1389 in the proximal drum portion 1383, securing joint rotation of the outer coil end portion 385a and the central rotor 1381. The second central spiral spring 386 has an outer coil end portion 386a which is inserted in a slot 1388 in the distal drum portion 1384, securing joint rotation of the outer coil end portion 386a and the central rotor 1381, and an inner coil end portion 386b which is rotationally fixed to the proximal shaft 383. Finally, the distal spiral spring 316 has an inner coil end portion 316b which is inserted in a slot 388 in the distal shaft 384, securing joint rotation of the inner coil end portion 316b and the distal rotor 381, and an outer coil end portion 316a adapted for rotational fixation to a portion of the drug delivery device which when rotated with respect to the stationary portion causes e.g. an expelling of a dose of the drug.

The proximal spiral spring 315 and the first central spiral spring 385 are wound in opposite directions about the proximal rotor 311. Similarly, the second central spiral spring 386 and the distal spiral spring 316 are wound in opposite directions about the distal rotor 381. Further, the first central spiral spring 385 and the second central spiral spring 386 have opposite winding directions. A pre-tensed spring assembly 310 will thus when released provide a total angular displacement of the outer coil end portion 316a relative to the outer coil end portion 315a which equals the sum of the relative angular displacements between the coil end portions of each individual spiral spring.

As can be seen, in principle, the spring assembly 310 comprises two sub-assemblies of the type shown in Fig. 3 arranged in series via the drum 1381, and it can as such directly replace the spring assembly 10 in an injection device of the type shown in Fig. 1 to provide for the emptying of a larger volume reservoir.

It is noted that all of the herein disclosed spring assemblies are capable of being extended as desired to meet specific requirements to the drug delivery device in which they are employed. The spring assembly 10 is e.g. extendable in the manner described with respect to Fig. 6, the spring assembly 210 is e.g. extendable by the addition of a further like rotor, carrying a further spiral spring in its drum portion, to the inner coil end portion 216, and so forth, and the spring assembly 310 is e.g. extendable by the addition of a further like drum, carrying a further like sub-assembly, to the outer coil end portion 316a, and so forth.

## Claims

1. A drug delivery device (1) comprising:
- a first interface portion (45),
- a second interface portion (22), and
- a power unit (10, 110, 210, 310) adapted to release energy for inducing relative rotation between the first interface portion (45) and the second interface portion (22),
**characterised in that** the power unit (10, 110, 210, 310) comprises:
∘ a first clock spring (15, 115, 215, 315) comprising a first coil end portion (15a, 115a, 215a, 315a) and a second coil end portion (15b, 115b, 215b, 315b), the first coil end portion (15a, 115a, 215a, 315a) being rotationally fixed to the first interface portion (45),
∘ a second clock spring (16, 116, 216, 316) comprising a third coil end portion (16b, 116b, 216a, 316b) and a fourth coil end portion (16a, 116a, 216b, 316a), the fourth coil end portion (16a, 116a, 216b, 316a) being rotationally fixed to the second interface portion (22), and
∘ an axially extending structure (11; 111; 211, 285, 281; 311, 385, 1381, 386, 381) operatively connecting the first clock spring (15, 115, 215, 315) and the second clock spring (16, 116, 216, 316),
wherein the second coil end portion (15b, 115b, 215b, 315b) and the third coil end portion (16b, 116b, 216a, 316b) are rotationally fixed to the axially extending structure (11; 111; 211, 285, 281; 311, 385, 1381, 386, 381) at different axial positions.

2. A drug delivery device according to claim 1, wherein the second coil end portion (15b, 115b, 215b) and the third coil end portion (16b, 116b, 316b) are respective inner coil end portions, and the first coil end portion (15a, 115a, 215a) and the fourth coil end portion (16a, 116a, 316a) are respective outer coil end portions.

3. A drug delivery device according to claim 1 or 2, wherein the first clock spring (15, 115) and the second clock spring (16, 116) are wound in opposite directions about the axially extending structure (11, 111).

4. A drug delivery device according to any of claims 1 - 3, wherein the axially extending structure (11) is torsionally rigid and comprises a first shaft portion (13) to which the second coil end portion (15b) is attached, and a second shaft portion (14) to which the third coil end portion (16b) is attached.

5. A drug delivery device according to any of claims 1 - 3, wherein the first clock spring (115), the second clock spring (116), and the axially extending structure (111) are formed from a single piece of material.

6. A drug delivery device according to claim 5, wherein the single piece of material comprises a bendable metal sheet having an axially extending central portion and two lateral strips extending from opposite sides of the central portion.

7. A drug delivery device according to claim 1 or 2, wherein the axially extending structure (211, 285, 281; 311, 385, 1381, 386, 381) comprises a first anchor part (211, 311) to which the second coil end portion (215b, 315b) is rotationally fixed, and a second anchor part (281, 381) to which the third coil end portion (216a, 316b) is rotationally fixed, and wherein the first anchor part (211, 311) and the second anchor part (281, 381) are rotatable relative to one another and biased to undergo relative rotation by at least one further clock spring (285; 385, 1381, 386).

8. A drug delivery device according to claim 7, wherein the at least one further clock spring (385, 1381, 386) comprises a third clock spring (385) comprising a fifth coil end portion (385a) and a sixth coil end portion (385b), a fourth clock spring (386) comprising a seventh coil end portion (386a) and an eighth coil end portion (386b), and a third anchor part (1381) being rotatable relative to both the first anchor part (311) and the second anchor part (381), the sixth coil end portion (385b) being rotationally fixed to the first anchor part (311), the eighth coil end portion (386b) being rotationally fixed to the second anchor part (381), and the fifth coil end portion (385a) and the seventh coil end portion (386a) being rotationally fixed to the third anchor part (1381) at different axial positions.

9. A drug delivery device according to any of the preceding claims, further comprising an expelling mechanism for delivering a dose of substance from a reservoir (70), the expelling mechanism comprising an actuator (6) for reducing a volume of the reservoir (70), wherein the second interface portion (22) is a portion of the expelling mechanism which when undergoing relative rotation with respect to the first interface portion (45) causes the actuator (6) to reduce the volume of the reservoir (70).

10. A drug delivery device according to claim 9, further comprising a housing (2) for accommodating at least a portion of the power unit (10, 110, 210, 310), wherein the first interface portion (45) is a portion of the housing (2), or is rotationally fixed with respect to the housing (2) at least when the power unit (10, 110, 210, 310) releases energy.

11. A drug delivery device according to claim 10, further comprising a dose setting mechanism comprising a dose dial (40) adapted for rotation relative to the housing (2) during setting of a dose and for rotational fixation with respect to the housing (2) during expelling of a set dose, wherein the first interface portion (45) is a portion of the dose dial (40).

12. A drug delivery device according to claim 11, wherein at least a portion of the power unit (10, 110, 210, 310) is contained within the dose dial (40).

13. A drug delivery device according to claim 12, further comprising an activation button (50) for activating the expelling mechanism, wherein the axially extending structure (11; 111; 211, 285, 281; 311, 385, 1381, 386, 381) forms a passage (17, 117), and wherein an inner portion (52) of the activation button (50) extends through the passage (17, 117), thereby constituting a hub for the power unit (10, 110, 210, 310).

14. A drug delivery device according to claim 13, wherein the second interface portion (22) is axially fixed with respect to the activation button (50), and wherein the injection button (50) is movable relative to the housing (2) between a proximal position in which the second interface portion (22) is rotationally fixed to the dose dial (40) and a distal position in which the second interface portion (22) is rotationally decoupled from the dose dial (40).

## Patentansprüche

1. Arzneimittelabgabevorrichtung (1), umfassend:
- einen ersten Grenzflächenabschnitt (45),
- einen zweiten Grenzflächenabschnitt (22), und
- eine Stromversorgungseinheit (10, 110, 210, 310) so angepasst, dass sie Energie für die Veranlassung relativer Drehung zwischen dem ersten Grenzflächenabschnitt (45) und dem zweiten Grenzflächenabschnitt (22) freisetzt,
**dadurch gekennzeichnet, dass** die Stromversorgungseinheit (10, 110, 210, 310) Folgendes umfasst:
∘ eine erste Spiralfeder (15, 115, 215, 315), die einen ersten Spulenendabschnitt (15a, 115a, 215a, 315a) und einen zweiten Spulenendabschnitt (15b, 115b, 215b, 315b) umfasst, wobei der erste Spulenendabschnitt (15a, 115a, 215a, 315a) am ersten Schnittstellenabschnitt (45) drehbar fixiert ist,
∘ eine zweite Spiralfeder (16, 116, 216, 316), die einen dritten Spulenendabschnitt (16b, 116b, 216a, 316b) und einen vierten Spulenendabschnitt (16a, 116a, 216b, 316a) umfasst, wobei der vierte Spulenendabschnitt (16a, 116a, 216b, 316a) am zweiten Schnittstellenabschnitt (22) drehbar fixiert ist, und
∘ eine sich axial erstreckende Struktur (11; 111; 211, 285, 281; 311, 385, 1381, 386, 381), die mit der ersten Spiralfeder (15, 115, 215, 315) und der zweiten Spiralfeder (16, 116, 216, 316) operativ verbunden ist,
worin der zweite Spulenendabschnitt (15b, 115b, 215b, 315b) und der dritte Spulenendabschnitt (16b, 116b, 216a, 316b) mit der sich axial erstreckenden Struktur (11; 111; 211, 285, 281; 311, 385, 1381, 386, 381) an verschiedenen Positionen drehbar fixiert sind.

2. Arzneimittelabgabevorrichtung nach Anspruch 1, worin der zweite Spulenendabschnitt (15b, 115b, 215b) und der dritte Spulenendabschnitt (16b, 116b, 316b) jeweilige innere Spulenendabschnitte sind, und der erste Spulenendabschnitt (15a, 115a, 215a) und der vierte Spulenendabschnitt (16a, 116a, 316a) jeweilige äußere Spulenendabschnitte sind.

3. Arzneimittelabgabevorrichtung nach Anspruch 1 oder 2, worin die erste Spiralfeder (15, 115) und die zweite Spiralfeder (16, 116) in entgegengesetzte Richtungen um die sich axial erstreckende Struktur (11, 111) gewickelt sind.

4. Arzneimittelabgabevorrichtung nach einem der Ansprüche 1 - 3, worin die sich axial erstreckende Struktur (11) torsionssteif ist und einen ersten Wellenabschnitt (13) umfasst, an dem der zweite Spulenendabschnitt (15b) angebracht ist, und einen zweiten Wellenabschnitt (14), an dem der dritte Spulenendabschnitt (16b) angebracht ist.

5. Arzneimittelabgabevorrichtung nach einem der Ansprüche 1 - 3, worin die erste Spiralfeder (115), die zweite Spiralfeder (116) und die sich axial erstreckende Struktur (111) aus einem einzigen Stück Material ausgebildet werden.

6. Arzneimittelabgabevorrichtung nach Anspruch 5, worin das einzige Stück Material ein biegbares Metallblech umfasst, das einen sich axial erstreckenden zentralen Abschnitt und zwei Seitenstreifen aufweist, die sich von entgegengesetzten Seiten des zentralen Abschnitts erstrecken.

7. Arzneimittelabgabevorrichtung nach Anspruch 1 oder 2, worin die sich axial erstreckende Struktur (211, 285, 281; 311, 385, 1381, 386, 381) einen ersten Ankerteil (211, 311) umfasst, an dem der zweite Spulenendabschnitt (215b, 315b) drehbar fixiert ist, und einen zweiten Ankerteil (281, 381), an dem der dritte Spulenendabschnitt (216a, 316b) drehbar fixiert ist, und worin der erste Ankerteil (211, 311) und der zweite Ankerteil (281, 381) gegeneinander drehbar und vorgespannt sind, um eine relative Drehung durch mindestens eine weitere Spiralfeder (285; 385, 1381, 386) durchzuführen.

8. Arzneimittelabgabevorrichtung nach Anspruch 7, worin die mindestens eine weitere Spiralfeder (385, 1381, 386) eine dritte Spiralfeder (385) umfasst, die einen fünften Spulenendabschnitt (385a) und einen sechsten Spulenendabschnitt (385b) umfasst, eine vierte Spiralfeder (386), die einen siebten Spulenendabschnitt (386a) und einen achten Spulenendabschnitt (386b) umfasst, und einen dritten Ankerteil (1381), der sowohl in Bezug auf den ersten Ankerteil (311) als auch den zweiten Ankerteil (381) drehbar ist, wobei der sechste Spulenendabschnitt (385b) am ersten Ankerteil (311) drehbar fixiert ist, wobei der achte Spulenendabschnitt (386b) am zweiten Ankerteil (381) drehbar fixiert ist, und der fünfte Spulenendabschnitt (385a) und der siebte Spulenendabschnitt (386a) am dritten Ankerteil (1381) an verschiedenen axialen Positionen drehbar fixiert sind.

9. Arzneimittelabgabevorrichtung nach einem der vorstehenden Ansprüche, ferner umfassend einen Ausstoßmechanismus für das Abgeben einer Dosis der Substanz aus einem Behälter (70), wobei der Ausstoßmechanismus ein Stellglied (6) für das Verringern eines Volumens des Behälters (70) umfasst, worin der zweite Grenzflächenabschnitt (22) ein Abschnitt des Ausstoßmechanismus ist, der bei Durchführung der relativen Drehung in Bezug auf den ersten Grenzflächenabschnitt (45) das Stellglied (6) veranlasst, das Volumen des Behälters (70) zu verringern.

10. Arzneimittelabgabevorrichtung nach Anspruch 9, ferner umfassend ein Gehäuse (2) für die Unterbringung mindestens eines Teils der Stromversorgungseinheit (10, 110, 210, 310), worin der erste Grenzflächenabschnitt (45) ein Abschnitt des Gehäuses (2) ist oder in Bezug auf das Gehäuse (2) drehbar fixiert ist, zumindest wenn die Stromversorgungseinheit (10, 110, 210, 310) Energie freisetzt.

11. Arzneimittelabgabevorrichtung nach Anspruch 10, ferner umfassend einen Dosiseinstellungsmechanismus (40), der für die Drehung in Bezug auf das Gehäuse (2) während der Einstellung einer Dosis und für die drehbare Fixierung in Bezug auf das Gehäuse (2) während des Ausstoßens einer festgelegten Dosis angepasst ist, worin der erste Grenzflächenabschnitt (45) ein Abschnitt des Dosierrads (40) ist.

12. Arzneimittelabgabevorrichtung nach Anspruch 11, worin mindestens ein Abschnitt der Stromversorgungseinheit (10, 110, 210, 310) im Dosierrad (40) enthalten ist.

13. Arzneimittelabgabevorrichtung nach Anspruch 12, ferner umfassend eine Aktivierungstaste (50) für das Aktivieren des Ausstoßmechanismus, worin die sich axial erstreckende Struktur (11; 111; 211, 285, 281; 311, 385, 1381, 386, 381) einen Durchgang (17, 117) ausbildet, und worin ein innerer Abschnitt (52) der Aktivierungstaste (50) sich durch den Durchgang (17, 117) erstreckt und dadurch einen Knotenpunkt für die Stromversorgungseinheit (10, 110, 210, 310) darstellt.

14. Arzneimittelabgabevorrichtung nach Anspruch 13, worin der zweite Grenzflächenabschnitt (22) in Bezug auf die Aktivierungstaste (50) axial fixiert ist, und worin die Injektionstaste (50) in Bezug auf das Gehäuse (2) zwischen einer proximalen Position, in der der zweite Grenzflächenabschnitt (22) an dem Dosierrad (40) drehbar fixiert ist, und einem distalen Abschnitt, in dem der zweite Grenzflächenabschnitt (22) von dem Dosierrad (40) drehentkoppelt ist, beweglich ist.

## Revendications

1. Dispositif d'administration de médicaments (1) comprenant
- une première partie d'interface (45),
- une deuxième partie d'interface (22), et
- une unité d'alimentation (10, 110, 210, 310) adaptée pour libérer de l'énergie afin d'induire une rotation relative entre la première partie d'interface (45) et la deuxième partie d'interface (22),
**caractérisé en ce que** l'unité d'alimentation (10, 110, 210, 310) comprend :
∘ un premier ressort d'horloge (15, 115, 215, 315) comprenant une première partie d'extrémité de bobine (15a, 115a, 215a, 315a) et une deuxième partie d'extrémité de bobine (15b, 115b, 215b, 315b), la première partie d'extrémité de bobine (15a, 115a, 215a, 315a) étant fixée de manière pivotante à la première partie d'interface (45),
∘ un deuxième ressort d'horloge (16, 116, 216, 316) comprenant une troisième partie d'extrémité de bobine (16b, 116b, 216a, 316b) et une quatrième partie d'extrémité de bobine (16a, 116a, 216b, 316a), la quatrième partie d'extrémité de bobine (16a, 116a, 216b, 316a) étant fixée de manière pivotante à la deuxième partie d'interface (22), et
∘ une structure s'étendant de manière axiale (11; 111; 211, 285, 281; 311, 385, 1381, 386, 381) connectant de manière fonctionnelle le premier ressort d'horloge (15, 115, 215, 315) et le deuxième ressort d'horloge (16, 116, 216, 316),
dans lequel la deuxième partie d'extrémité de bobine (15b, 115b, 215b, 315b) et la troisième partie d'extrémité de bobine (16b, 116b, 216a, 316b) sont fixées de manière pivotante à la structure s'étendant de manière axiale (11; 111; 211, 285, 281; 311, 385, 1381, 386, 381) à des positions axiales différentes.

2. Dispositif d'administration de médicaments selon la revendication 1, dans lequel la deuxième partie d'extrémité de bobine (15b, 115b, 215b) et la troisième partie d'extrémité de bobine (16b, 116b, 316b) sont des parties d'extrémité de bobine internes respectives, et la première partie d'extrémité de bobine (15a, 115a, 215a) et la quatrième partie d'extrémité de bobine (16a, 116a, 316a) sont des parties d'extrémité de bobine externes respectives.

3. Dispositif d'administration de médicaments selon la revendication 1 ou 2, dans lequel le premier ressort d'horloge (15, 115) et le deuxième ressort d'horloge (16, 116) sont enroulés dans des directions opposées autour de la structure s'étendant de manière axiale (11, 111).

4. Dispositif d'administration de médicaments selon l'une quelconque des revendications 1 - 3, dans lequel la structure s'étendant de manière axiale (11) présente une rigidité en torsion et comprend une première partie arbre (13) à laquelle la deuxième partie d'extrémité de bobine (15b) est attachée, et une deuxième partie arbre (14) à laquelle la troisième partie d'extrémité de bobine (16b) est attachée.

5. Dispositif d'administration de médicaments selon l'une quelconque des revendications 1 - 3, dans lequel le premier ressort d'horloge (115), le deuxième ressort d'horloge (116), et la structure s'étendant de manière axiale (111) sont formés d'une unique pièce de matériau.

6. Dispositif d'administration de médicaments selon la revendication 5, dans lequel l'unique pièce de matériau comprend une feuille métallique flexible ayant une partie centrale s'étendant de manière axiale et deux bandes latérales s'étendant depuis les côtés opposés de la partie centrale.

7. Dispositif d'administration de médicaments selon la revendication 1 ou 2, dans lequel la structure s'étendant de manière axiale (211, 285, 281; 311, 385, 1381, 386, 381) comprend une première partie d'ancrage (211, 311) à laquelle la deuxième partie d'extrémité de bobine (215b, 315b) est fixée de manière pivotante, et une deuxième partie d'ancrage (281, 381) à laquelle la troisième partie d'extrémité de bobine (216a, 316b) est fixée de manière pivotante, et dans lequel la première partie d'ancrage (211, 311) et la deuxième partie d'ancrage (281, 381) peuvent pivoter l'une par rapport à l'autre et être déplacées pour subir une rotation relative par au moins un ressort supplémentaire d'horloge (285; 385, 1381, 386).

8. Dispositif d'administration de médicaments selon la revendication 7, dans lequel l'au moins un ressort supplémentaire d'horloge (385, 1381, 386) comprend un troisième ressort d'horloge (385) comprenant une cinquième partie d'extrémité de bobine (385a) et une sixième partie d'extrémité de bobine (385b), un quatrième ressort d'horloge (386) comprenant une septième partie d'extrémité de bobine (386a) et une huitième partie d'extrémité de bobine (386b), et une troisième partie d'ancrage (1381) pouvant être pivotée par rapport à, à la fois, la première partie d'ancrage (311) et la deuxième partie d'ancrage (381), la sixième partie d'extrémité de bobine (385b) étant fixée de manière pivotante à la première partie d'ancrage (311), la huitième partie d'extrémité de bobine (386b) étant fixée de manière pivotante à la deuxième partie d'ancrage (381), et la cinquième partie d'extrémité de bobine (385a) et la septième partie d'extrémité de bobine (386a) étant fixée de manière pivotante à la troisième partie d'ancrage (1381) à des positions axiales différentes.

9. Dispositif d'administration de médicaments selon l'une quelconque des revendications précédentes, comprenant en outre un mécanisme d'expulsion pour l'administration d'une dose de substance depuis un réservoir (70), le mécanisme d'expulsion comprenant un actionneur (6) pour réduire le volume du réservoir (70), dans lequel la deuxième partie d'interface (22) est une partie du mécanisme d'expulsion qui, lorsqu'il est soumis à une rotation relative par rapport à la première partie d'interface (45), amène l'actionneur (6) à réduire le volume du réservoir (70).

10. Dispositif d'administration de médicaments selon la revendication 9, comprenant en outre un boîtier (2) destiné à recevoir au moins une partie de l'unité d'alimentation (10, 110, 210, 310), dans lequel la première partie d'interface (45) est une partie du boîtier (2), ou est fixée de manière pivotante par rapport au boîtier (2) au moins lorsque l'unité d'alimentation (10, 110, 210, 310) libère de l'énergie.

11. Dispositif d'administration de médicaments selon la revendication 10, comprenant en outre un mécanisme de réglage de dose comprenant une sélection de dose (40) adaptée pour pivoter autour du boîtier (2) pendant le réglage d'une dose et pour une fixation rotationnelle par rapport au boîtier (2) lors de l'expulsion d'une dose définie, dans lequel la première partie d'interface (45) est une partie de la sélection de dose (40).

12. Dispositif d'administration de médicaments selon la revendication 11, dans lequel au moins une partie de l'unité d'alimentation (10, 110, 210, 310) est contenue dans la sélection de dose (40).

13. Dispositif d'administration de médicaments selon la revendication 12, comprenant en outre un bouton d'activation (50) pour activer le mécanisme d'expulsion, dans lequel la structure s'étendant de manière axiale (11; 111; 211, 285, 281; 311, 385, 1381, 386, 381) forme un passage (17, 117), et dans lequel une partie interne (52) du bouton d'activation (50) s'étend à travers le passage (17, 117), constituant ainsi un centre pour l'unité d'alimentation (10, 110, 210, 310).

14. Dispositif d'administration de médicaments selon la revendication 13, dans lequel la deuxième partie d'interface (22) est fixée de manière axiale par rapport au bouton d'activation (50), et dans lequel le bouton d'injection (50) est mobile par rapport au boîtier (2) entre une position proximale où la deuxième partie d'interface (22) est fixée de manière pivotante à la sélection de dose (40) et une position distale où la deuxième partie d'interface (22) est découplée par rotation de la sélection de dose (40).
